# EUROPÄISCHE PATENTSCHRIFT

(11) **EP 3 079 729 B1**
(45) Veröffentlichungstag und Bekanntmachung des Hinweises auf die Patenterteilung: **27.06.2018**
(21) Anmeldenummer: 14841332.1
(22) Anmeldetag: 11.12.2014
(51) Int. Cl.: A61L 2/14, A61N 1/04, H05H 1/46

(54) **ANORDNUNG ZUR BEHANDLUNG VON WUNDEN**
ASSEMBLY FOR THE TREATMENT OF WOUNDS
DISPOSITIF POUR TRAITER DES PLAIES

(30) Priorität: 12.12.2013 DE 102013113905
(43) Veröffentlichungstag der Anmeldung: 19.10.2016
(73) Patentinhaber: Relyon Plasma GmbH, 93057 Regensburg (DE)
(72) Erfinder: NETTESHEIM, Stefan, 93051 Regensburg (DE); KORZEC, Dariusz, 93173 Wenzenbach (DE); BURGER, Dominik, 93087 Alteglofsheim (DE)
(74) Vertreter: Reichert & Lindner Partnerschaft Patentanwälte
(86) Internationale Anmeldenummer: PCT/IB2014/066808
(87) Internationale Veröffentlichungsnummer: WO 2015/087278

(56) Entgegenhaltungen:
- EP-A1- 2 170 022
- EP-A2- 2 599 506
- DE-A1-102011 001 416
- US-A1- 2013 226 073

## Beschreibung

Die Erfindung betrifft eine Anordnung zur Behandlung von Wunden. Das Gerät zur Erzeugung eines Plasmas oder eines angeregten Gases bzw. Gasgemisches weist ein Gehäuse auf, wobei durch das Gerät in einem Bereich einer Öffnung des Gehäuses das Plasma oder das angeregte Gas bzw. Gasgemisch erzeugbar ist.

Die noch nicht veröffentlichte Patentanmeldung DE 10 2013 107 448.0 offenbart eine Vorrichtung zur Keimreduktion mittels Plasma. Die dielektrische Folie schließt mit ihrem umlaufenden Rand einen zu entkeimenden Bereich (Wunde oder Gegenstand) ein. Ein Hochspannungsende des piezoelektrischen Transformators ist der Außenseite der dielektrischen Folie zugewandt und das Plasma innerhalb der dielektrischen Folie gezündet.

Die internationale Patentanmeldung WO 2010/034451 A1 offenbart einen Plasma-Applikator zum Anlegen eines nicht-thermischen Plasmas auf eine Oberfläche, insbesondere für die Plasmabehandlung von lebendem Gewebe und insbesondere für die Plasmabehandlung von Wunden. Das Plasma-Behandlungsgerät hat einen Verschlussdeckel zum Ab-/Überdecken eines Abschnitts der Oberfläche. Dadurch wird ein Hohlraum zwischen dem Verschlussdeckel und der Oberfläche Das nicht-thermische Plasma ist im Hohlraum vorgesehen und zusätzlich kann der Hohlraum mit Gas gespült werden. Ebenso ist eine Pumpe vorgesehen, die Gas aus dem Hohlraum absaugt.

Die internationale Patentanmeldung WO 2013/093868 A1 offenbart eine Vorrichtung zur selektiven Aktivierung für medizinische Implantate mittels Plasma und eine Vorrichtung zur Wundheilung. Von einem Plasmagenerator wird das Plasma mittels eines flexiblen Schlauchs zu einem beweglichen Applikator oder Kopf geleitet. Das Plasma wird über Öffnungen in einer Schablone auf ein Implantat gerichtet.

Die deutsche Patentanmeldung DE 10 2011 001 416 A1 offenbart eine Plasmabehandlungseinrichtung zur Behandlung von Wunden oder erkrankten Hautpartien. Die Plasmabehandlungseinrichtung weist zwei flexible Flächenelektroden zur Erzeugung eines nicht-thermischen Plasmas auf. Die beiden Flächenelektroden bestehen jeweils aus mindestens einem elektrischen Leiter, wobei die Leiter miteinander verwoben sind. Auf der, der zu behandelnden Oberfläche zugewandten, Außenseite der Flächenelektroden ist eine Wundkontaktschicht aus einem antiseptisch behandelten Material lösbar befestigt. Aus EP 2 599 506 A2 ist eine Plasmabehandlungseinrichtung bekannt, bei der das Plasma von Impedanzwandlern auf Basis einer nichtionisierenden Mikrowellenstrahlungsquelle in Kombination mit einem Gas erzeugt wird.

Der Erfindung liegt die Aufgabe zugrunde, eine Anordnung zur Behandlung von Wunden zu schaffen, die kostengünstig ist, einfach anzuwenden ist und mit der eine effiziente und sichere Behandlung von Wunden gegeben ist.

Die obige Aufgabe wird mit einer Anordnung gemäß den Merkmalen des Anspruchs 1 gelöst.

Die erfindungsgemäße Anordnung zur Behandlung von Wunden umfasst ein Gerät, das ein Gehäuse aufweist. Mit dem Gerät kann in einem Bereich einer Öffnung des Gehäuses ein Plasma oder ein angeregtes Gas bzw. Gasgemisch erzeugt werden. Eine erste Leitung für das Plasma oder das angeregte Gas bzw. Gasgemisch führt zu einer Abdeckung für den zu behandelnden Wundbereich. Eine zweite Leitung führt von der Abdeckung für den zu behandelnden Wundbereich zu einer Pumpe. Die Abdeckung für den zu behandelnden Wundbereich ist mit ihrem umlaufenden Rand mit dem Körperteil, sprich der Haut, verklebt bzw. derart verbunden, dass der umlaufende Rand der Abdeckung den zu behandelnden Wundbereich umschließt. Zwischen der Pumpe und dem Gerät kann eine elektronische Kopplung vorgesehen sein. Die Kopplung kann mittels einer Leitung oder drahtlos erfolgen und hat den Vorteil, dass auf einfache Weise die Pumpe und das Gerät derart aufeinander abgestimmt werden können, dass eine effektive Wundbehandlung durchgeführt werden kann. Ebenso werden unnötige Belastungen des Patienten durch eine zu lange oder zu hoch dosierte Behandlung mit dem Plasma oder dem angeregten Gas bzw. Gasgemisch vermieden. Falls mit dem Gerät ein Plasma erzeugt wird, kann mit dem Gerät Raumluft direkt oder über einen Filter angesaugt werden.

Ebenso kann über eine Leitung dem Gerät zur Erzeugung eines Plasmas oder eines angeregten Gases bzw. Gasgemisches, Prozessgas definiert zugeführt werden. Das Gerät kann ein Plasmagenerator oder lonisator oder Ozonisator sein. Für den Fall, dass das Gerät eine lonisator oder Ozonisator ist wirkt anstelle eines Plasmas ein Gasgemisch mit angeregten Molekülen, Ionen und reaktiven Sauerstoffspezies, wie z.B. Ozon, atomarer Sauerstoff, H2O2, OH-Radikale oder NOx, auf den Wundbereich ein.

Streng genommen muss nicht unbedingt ein Plasma den Wundbereich erreichen.

Das im Gerät erzeugte Plasma oder das angeregte Gas bzw. Gasgemisch wird mittels einer ersten Leitung zu der Abdeckung geführt. Die Abdeckung hat mindestens einen Zuleitport und mindestens einen Ableitport an der Abdeckung ausgebildet. Die erste Leitung wird hierzu mit dem Zuleitport und die zweite Leitung, zu der Pumpe, wird mit dem Ableitport verbunden. Die erste Leitung besitzt ein Anschlusselement, das mit der Öffnung des Gehäuses lösbar verbindbar ist. Somit gelangt das Plasma oder das angeregte Gas bzw. Gasgemisch direkt vom Gerät in die erste Leitung.

Bei der Abdeckung ist zwischen der Abdeckung und dem zu behandelnden Wundbereich eine Wundauflage vorgesehen. Zur gerichteten Führung des Plasmas oder des angeregten Gases bzw. Gasgemisches durch die Wundauflage und zur effizienten Einwirkung auf den zu behandelnden Wundbereich ist innerhalb der Wundauflage ein Mittel vorgesehen, mit dem eine Zwangsführung des Plasmas oder des angeregten Gases bzw. Gasgemisches vom Zuleitport zum Ableitport erzielt wird. Das Plasma oder das angeregte Gas bzw. Gasgemisch wird dabei auf einem Weg vom Zuleitport zum Ableitport über bzw. an den zu behandelnden Wundbereich geführt.

Das Mittel zur Zwangsführung des Plasmas oder des angeregten Gas bzw. Gasgemisch kann in unterschiedlichen Ausführungen ausgestaltet sein. So kann das Mittel zur Zwangsführung des Plasmas oder des angeregten Gases bzw. Gasgemisches, z.B. mehrere gerade Verteilkanäle umfassen die von einem Verteilelement zu einem Sammelelement führen. Das Verteilelement ist mit dem Zuleitport und das Sammelelement ist mit dem Ableitport verbunden. Die mehreren geraden Leitungen sind derart in die Wundauflage eingebettet, dass aus den Verteilkanälen das austretende Plasma oder das angeregte Gas bzw. Gasgemisch auf die zu behandelnde Wunde einwirkt.

Gemäß einer weiteren Ausführungsform ist das Mittel zur Zwangsführung des Plasmas oder des angeregten Gases bzw. Gasgemisches als ein einziger mäandernder Verteilkanal ausgebildet, der direkt mit dem Zuleitport und dem Ableitport verbunden ist.

Eine weitere Möglichkeit für das Mittel zur Zwangsführung des Plasmas des angeregten Gases bzw. Gasgemisches sind mehrere Strömungshindernisse. Die Strömungshindernisse sind in der Wundauflage gemäß einem regelmäßigen Gitter verteilt und sind zwischen einem Verteilelement und einem Sammelelement angeordnet. Das Verteilelement ist mit dem Zuleitport und das Sammelelement mit dem Ableitport verbunden. Das Plasma oder das angeregte Gas bzw. Gasgemisch tritt aus dem Verteilelement und bewegt sich aufgrund der Pumpwirkung am Sammelelement durch die Wundauflage. Die Strömungshindernisse haben den Vorteil, dass sie für eine Gleichverteilung des Plasmas oder des angeregten Gases bzw. Gasgemisches in der Wundauflage sorgen.

Eine weitere Möglichkeit für das Mittel zur Zwangsführung des Plasmas oder des angeregten Gases bzw. Gasgemisches sind mehrere Verteilkanäle, die mit jeweils zwei Umlenkungen versehen sind. Die Leitungen führen vom Verteilelement zum Sammelelement. Das Verteilelement ist wiederum mit dem Zuleitport und das Sammelelement ist mit dem Ableitport verbunden. Für den Fall, dass das Verteilelement und das Sammelelement außerhalb der Abdeckung angeordnet sind, werden wie aus der Abdeckung ragenden Leitungen mit dem Verteilelement bzw. dem Sammelelement verbunden.

Eine weitere Ausführungsform des Mittels zur Zwangsführung des Plasmas oder des angeregten Gases bzw. Gasgemisches ist als offenporige Struktur ausgebildet. Die offenporige Struktur ist auf den zu behandelnden Wundbereich hin ausgerichtet.

Die Wundauflage hat eine Dicke von z.B. 3 mm und die Verteilkanäle haben eine Tiefe von ca. 1,5 mm und sind zum Wundebereich hin offen. Typischerweise können die Verteilkanäle auch als Gasverteiler oder englisch "flow-field" bezeichnet werden. Anstelle des in geordneter Form strukturierten Gasverteilers kann der Gasverteiler auch eine zum Wundbereich hin offenporige Struktur sein.

Um die Verteilkanäle bzw. den Gasverteiler frei von Flüssigkeit zu halten, können diese hydrophob beschichtet sein. Eine besonders geeignete Ausführungsform wäre, die Gasverteilungskanäle in die Wundauflage einzuprägen.

Ein Farbsensor kann ebenfalls der Abdeckung zugeordnet sein, um über eine Farbänderung anzuzeigen, dass die Behandlung abgeschlossen ist. Ferner kann ein RFID-Chip der Abdeckung zugeordnet sein, so dass über den Typ der Abdeckung und in Verbindung mit Gerät und Pumpe zumindest eine Behandlungsdauer einstellbar ist. Mittels des RFID-Chips ist es möglich, das Gerät und die Pumpe derart abzustimmen, dass zumindest die Stärke und die Dauer des erzeugten Plasmas oder des angeregten Gases bzw. Gasgemisches und ggf. die Zuführung von zusätzlichem Gas (wie z.B. Argon) auf den Typ der Abdeckung abgestimmt sind.

Gemäß einer möglichen Ausführungsform ist das Gerät ein Plasmaerzeugungsgerät mit einem piezoelektrischen Transformator zur Erzeugung des Plasmas. Das Hochspannungsende des piezoelektrischen Transformators ist zur Öffnung im Gehäuse hin ausgerichtet. Das Plasmaerzeugungsgerät ist als Handgerät ausgebildet.

Es ist von Vorteil, dass das Gerät ein Handgerät ist. Das Handgerät ist kostengünstig und gewährleistet eine einfache Handhabung. In Verbindung mit der stationären Abdeckung kann trotz des Handgeräts eine sichere und effiziente Behandlung der Wunde eingehalten werden. Hinzu kommt, dass die Abdeckung, die Wundauflage und die Mittel zur Zwangsführung des Plasmas in einfacher und kostengünstiger Weise und in unterschiedlichen Ausstattungsvarianten hergestellt werden können.

Weitere Vorteile und vorteilhafte Ausgestaltungen der Erfindung sind Gegenstand der nachfolgenden Figuren, sowie deren Beschreibungsteile.

Es zeigen im Einzelnen:
- **Figur 1**: eine schematische Ansicht des prinzipiellen Aufbaus der erfindungsgemäßen Anordnung zur Plasmabehandlung von Wunden;
- **Figur 2**: eine mögliche Ausführung der Abdeckung mit einem Mittel zur Zwangsführung des Plasmas oder des angeregten Gases bzw. Gasgemisches über den zu behandelnden Wundbereich;
- **Figur 3**: eine mögliche weitere Ausführung der Abdeckung mit einem Mittel zur Zwangsführung des Plasmas oder des angeregten Gases bzw. Gasgemisches über den zu behandelnden Wundbereich;
- **Figur 4**: eine noch weitere Ausführung der Abdeckung mit einem Mittel zur Zwangsführung des Plasmas oder des angeregten Gases bzw. Gasgemisches über den zu behandelnden Wundbereich;
- **Figur 5**: eine zusätzlich weitere Ausführung der Abdeckung mit einem Mittel zur Zwangsführung des Plasmas oder des angeregten Gases bzw. Gasgemisches über den zu behandelnden Wundbereich; und
- **Figur 6**: eine Schnittansicht der Abdeckung für den Wundbereich, wobei die Zuordnung der Mittel zur Zwangsführung zum behandelnden Wundbereich zu erkennen ist.

Für gleiche oder gleich wirkende Elemente der Erfindung werden identische Bezugszeichen verwendet. Die dargestellten Ausführungsformen stellen lediglich eine Möglichkeit dar, wie die Anordnung zur Behandlung von Wunden mittels eines erzeugten Plasmas ausgestaltet sein kann. Obwohl sich die nachstehende Beschreibung ausschließlich auf ein Gerät bezieht, das als Plasmaerzeugungsgerät ausgebildet ist, soll dies nicht als Beschränkung der Erfindung aufgefasst werden. Wie bereits oben erwähnt, kann anstelle eines Plasmas auch ein Gasgemisch mit angeregten Molekülen, Ionen und reaktiven Sauerstoffspezies, wie z.B. Ozon, atomarer Sauerstoff, H2O2, OH-Radikale oder NOx die Wirkung bei der Behandlung von Wunden entfalten. Streng genommen muss auch nicht unbedingt Plasma den Wundbereich erreichen.

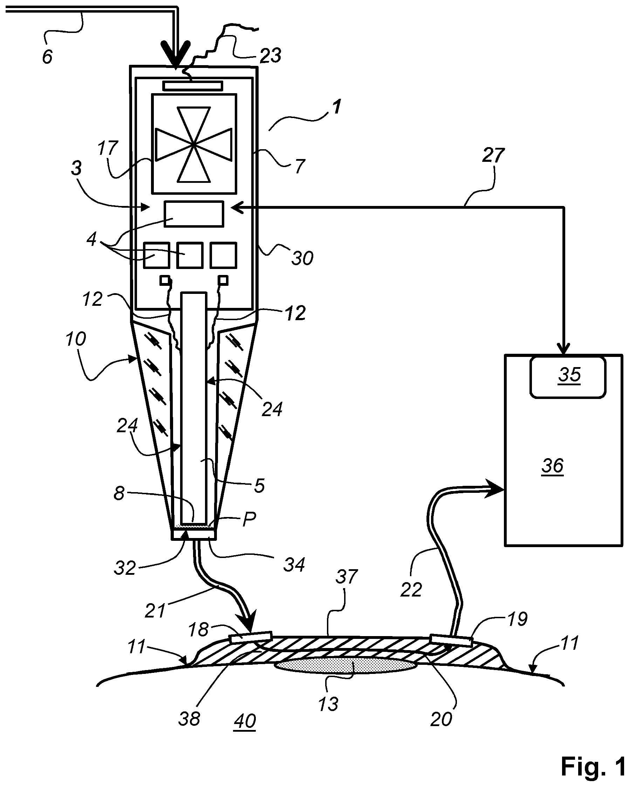

Eine schematische Ansicht der erfindungsgemäßen Anordnung 1 zur Behandlung von Wunden ist in **Figur 1** dargestellt. Bei der hier dargestellten Ausführungsform wird das Plasma P in einem Plasmaerzeugungsgerät 10 mit einem piezoelektrischen Transformator 5 erzeugt. Der piezoelektrische Transformator 5 ist in einem Gehäuse 30 des Plasmaerzeugungsgeräts 10 untergebracht. Es ist für einen Fachmann selbstverständlich, dass das Plasma P auch auf anderem Wege als mit einem piezoelektrischen Transformator 5 erzeugt werden kann. Obwohl sich die nachstehende Beschreibung auf einen piezoelektrischen Transformator 5 bezieht, soll dies nicht als eine Beschränkung der Erfindung aufgefasst werden. Zur Ansteuerung ist der piezoelektrische Transformator 5 mit einer Platine 7 verbunden. Die Platine 7 realisiert mit einer Vielzahl von elektronischen Bauelementen 4 eine Steuerschaltung 3. Mit der Steuerschaltung 3 ist es möglich, den piezoelektrischen Transformator 5 mit seiner Resonanzfrequenz anzuregen. Die Steuerschaltung 3 für den piezoelektrischen Transformator 5 kann mit einer externen Energieversorgung verbunden werden, die ein herkömmliches Standard-Netzteil (nicht dargestellt) ist, das über ein Kabel 23 mit dem Gehäuse 30 des piezoelektrischen Transformators 5 verbunden ist. Ebenso kann die Energieversorgung mit einem Akku durchgeführt werden. Eine Kombination aus Akku und Standard-Netzteil ist ebenfalls denkbar. Die Ansteuerspannung wird von der Steuerschaltung 3 der Platine 7 über je einen elektrischen Anschluss 12 an je eine Seitenfläche 24 des piezoelektrischen Transformators 5 angelegt. Durch die an den Seitenflächen 24 des piezoelektrischen Transformators 5 anliegende Anregungsspannung bildet sich am Hochspannungsende 8 des piezoelektrischen Transformators 5 die erforderliche Hochspannung aus. Im oder an dem Gehäuse 30 kann ferner ein Lüfter 17 vorgesehen sein, der im Gehäuse 30 für eine Luftströmung zur Öffnung 32 des Gehäuses 30 hin sorgt. Ebenso kann über eine Gasleitung 6 dem Plasmaerzeugungsgerät 10 Gas oder ein Gasgemisch zugeführt werden, mit dem über dem piezoelektrischen Transformator 5 das Plasma gezündet wird.

Ein zu behandelnder Wundbereich 13 ist mit einer Abdeckung 37 versehen, wobei die Abdeckung 37 mit einem umlaufenden Rand 11 den zu behandelnden Wundbereich 13 umschließt und mit einen Körperteil 40 an dem sich der zu behandelnde Wundbereich 13 befindet, verklebt ist. Zwischen der Abdeckung 37 und dem zu behandelnden Wundbereich 13 ist eine Wundauflage 38 vorgesehen. Ferner hat die Abdeckung 37 mindestens einen Zuleitport 18 und mindestens ein Ableitport 19 ausgebildet.

Das Plasma P wird vom Plasmaerzeugungsgerät 10 mittels einer ersten Leitung 21 zu dem Zuleitport 18 transportiert. Die erste Leitung 21 ist über ein Anschlusselement 34 mit dem Plasmaerzeugungsgerät 10 verbunden. Das Anschlusselement 34 ist hierzu lösbar mit der Öffnung 32 des Gehäuses 30 verbunden.

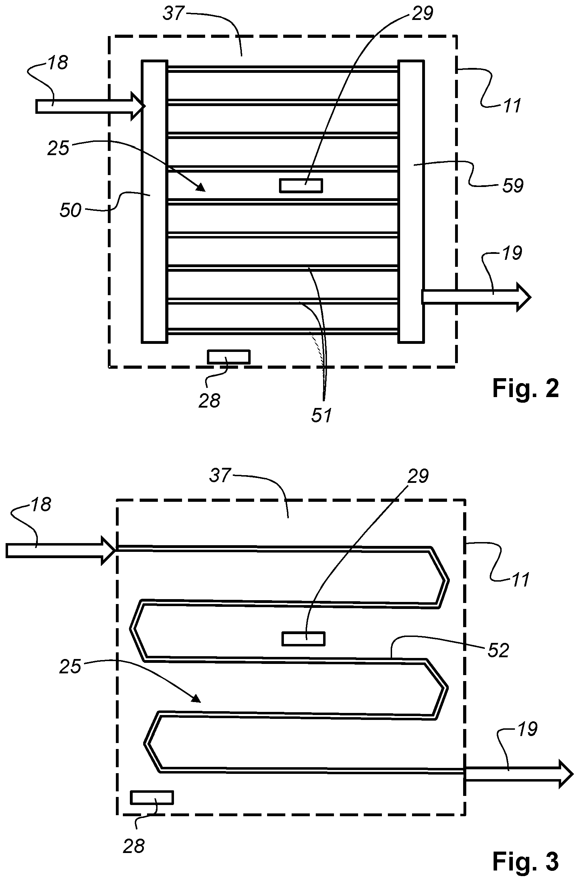

Die zweite Leitung 22 führt von mindestens einem Ableitport 19 der Abdeckung 37 zu einer Pumpe 36. Mittels der Pumpe 36 kann innerhalb der Wundauflage 38 ein gerichteter Strom des Plasmas P über den zu behandelnden Wundbereich 13 erzielt werden. Durch gezielte Abstimmung der Saugleistung, der Pumpe P und der Leistung des Plasmaerzeugungsgeräts 10 kann eine optimale und effiziente Behandlung des Wundbereichs 13 erzielt werden. Die gezielte Abstimmung kann durch eine Kopplung 27 der Pumpe 36 mit dem Plasmaerzeugungsgerät 10 erzielt werden. Die Kopplung 27 kann drahtlos oder über eine Leitung erfolgen. Die Steuerschaltung 35 der Pumpe 36 und die Steuerschaltung 3 des Plasmaerzeugungsgeräts 10 können somit eine gezielte Regulierung vornehmen, um eine effiziente und erfolgreiche Wundbehandlung zu erzielen. Hinzu kommt, dass innerhalb der Wundauflage Mittel (siehe Fig. 2 bis Fig. 5) vorgesehen sind, mit denen eine Zwangsführung des Plasmas P vom Zuleitport 18 zum Ableitport 19 bewirkt wird. Das Plasma P ist somit auf einem Weg vom Zuleitport 18 zum Ableitport 19 über den zu behandelnden Wundbereich 13 geführt.

In **Figur 2** ist eine mögliche Ausführung der Abdeckung 37 mit dem Mittel 25 zur Zwangsführung des Plasmas P. Das Mittel 25 zur Zwangsführung des Plasmas P ist innerhalb der Wundauflage 38 (siehe Figur 1) vorgesehen. Bei der in Figur 2 gezeigten Ausführungsform besteht das Mittel 25 zur Zwangsführung des Plasmas P aus mehreren geraden Leitungen 51. Die von einem Verteilelement 50 zu einem Sammelelement 59 führen. Das Verteilelement 50 ist dabei mit dem Zuleitport 18 und das Sammelelement 59 ist mit dem Ableitport (19) verbunden.

In **Figur 3** ist eine weitere mögliche Ausführung für das Mittel 25 zur Zwangsführung des Plasmas P offenbart. Es ist eine einzige mäandernde Leitung 52 vorgesehen, die direkt mit dem Zuleitport 18 und dem Ableitport 19 verbunden ist.

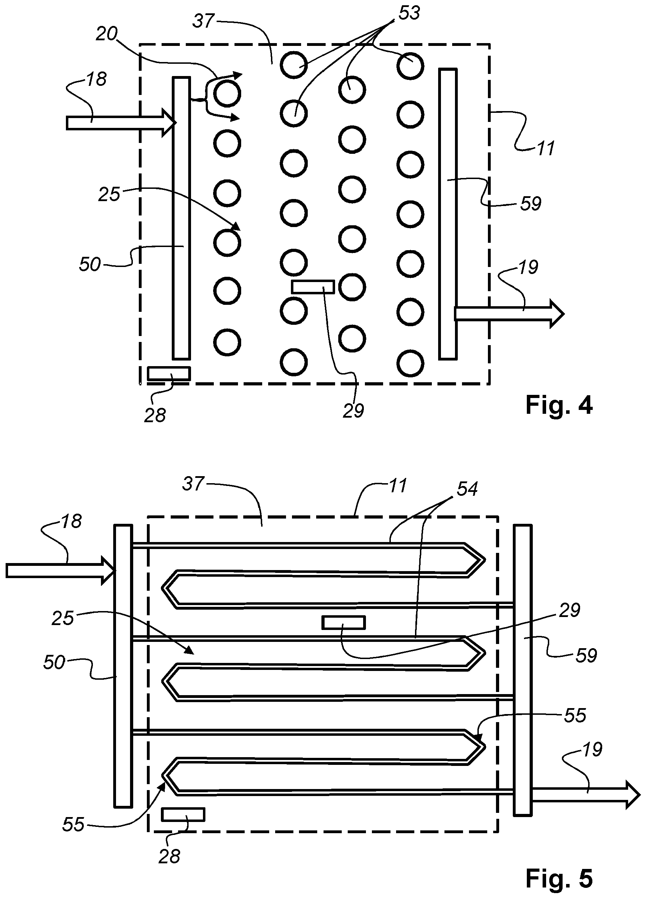

Bei der in **Figur 4** gezeugten Ausführungsform stellen mehrere Strömungshindernisse 53 das Mittel 25 zur Zwangsführung des Plasmas P dar. Die Strömungshindernisse 53 sind in einem regelmäßigen Gitter verteilt angeordnet und zwischen dem Verteilelement 50 und dem Sammelelement 59 vorgesehen. Das Verteilelement 50 ist mit dem Zuleitport 18 und das Sammelelement 59 mit dem Ableitport 19 verbunden. Aus dem Verteilelement 50 tritt das Plasma P aus und der Plasmastrom 20 verteilt sich gleichmäßig auf dem Weg zum Sammelelement 59.

Das Mittel 25 zur Zwangsführung des Plasmas P umfasst bei der in **Figur 5** gezeigten Ausführungsform mehrere Leitungen 54, die jeweils zwei Umlenkungen 55 ausgebildet haben. Die Leitungen 54 führen vom Verteilelement 50 zum Sammelelement 59. Auch hier ist das Verteilelement 50 mit dem Zuleitport 18 und das Sammelelement 59 mit dem Ableitport 19 verbunden.

Die Abdeckung 37 kann mit einem RFID-Chip 28 und/oder einem Farbsensor 29 versehen werden. Bei der Verwendung eines RFID-Chips 28 kann das Plasmaerzeugungsgerät 10 den Typ der Abdeckung 37 auslesen und über die Steuerschaltung 3 entsprechende Einstellungen des Plasmaerzeugungsgeräts 10 und/oder über die Kopplung 27 Einstellungen an der Pumpe 36 vornehmen. Der Farbsensor 29 kann über eine Farbänderung anzuzeigen, dass die Behandlung mit der erfindungsgemäßen Anordnung abgeschlossen ist. Es ist selbstverständlich, dass der RFID-Chip 28 und der Farbsensor 29 allein und/oder in Kombination mit der Abdeckung 37 verwendet werden können.

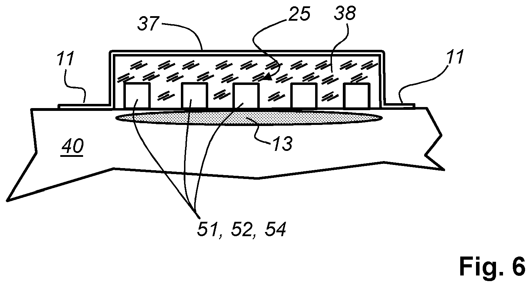

**Figur 6** zeigt eine Schnittansicht der Abdeckung 37 für den Wundbereich 13. Die Abdeckung 37 umgibt die Wundauflage 38 und ist mit dem Körperteil 40 über den umlaufenden Rand 11 verklebt, so dass der Wundbereich 13 umgeben ist und die Wundauflage 38 auf dem Wundbereich 13 liegt. Die Wundauflage 38 hat der Mittel zur Zwangsführung für das Plasma P ausgebildet, so dass das Plasma P oder aufgrund des Plasmas in Zusammenwirkung mit anderen Substanzen eine reaktives Gas mit dem zum behandelnden Wundbereich 13 in Wechselwirkung tritt. Die Wundauflage hat mindestens einen Verteilerkanal 51, 52, 54 ausgebildet. Die Verteilerkanäle 51, 52, 54 der Wundauflage 38 sind zum Wundbereich 13 hin offen. Um die Verteilerkanäle 51, 52, 54 frei von Flüssigkeit halten zu können, sind diese hydrophob beschichtet. Eine besonders geeignete Möglichkeit der Herstellung der Verteilerkanäle 51, 52, 54 ist, dass diese in die Wundauflage 38 eingeprägt werden.

### Bezugszeichenliste:

- 1: Anordnung
- 3: Steuerschaltung
- 4: elektronische Bauelemente
- 5: piezoelektrischer Transformator
- 6: Gasleitung
- 7: Platine
- 8: Hochspannungsende
- 10: Plasmaerzeugungsgerät
- 11: umlaufender Rand
- 12: elektrischer Anschluss
- 13: Wundbereich
- 14: Strömungsleitelement
- 15: Plasmaströmung
- 17: Lüfter
- 18: Zuleitport
- 19: Ableitport
- 20: Plasmastrom
- 21: erste Leitung
- 22: zweite Leitung
- 23: Kabel von Netzteil
- 24: Seite des piezoelektrischen Transformator
- 25: Mittel (Zwangsführung)
- 27: Kopplung
- 28: RFID-Chip
- 29: Farbsensor
- 30: Gehäuse
- 32: Öffnung
- 34: Anschlusselement
- 35: Steuerschaltung
- 36: Pumpe
- 37: Abdeckung
- 38: Wundauflage
- 40: Körperteil
- 50: Verteilelement
- 51: gerader Verteilerkanal
- 52: mäandernder Verteilerkanal
- 53: Strömungshindernis
- 54: Verteilkanal mit Umlenkung
- 59: Sammelelement
- P: Plasma

## Patentansprüche

1. Anordnung (1) zur Behandlung von Wunden umfassend
ein Gerät (10) zur Erzeugung eines Plasmas (P) oder eines angeregten Gases bzw. Gasgemisches;
eine Abdeckung (37) für den zu behandelnden Wundbereich (13); eine Pumpe (36); ein Gehäuse (30) des Geräts (19);
**dadurch gekennzeichnet, dass**
die Anordnung (1) eine erste Leitung (21) zum Zuführen des Plasmas (P) oder des angeregten Gasgemisches vom Gerät (10) zur Abdeckung (37) für den zu behandelnden Wundbereich (13);
eine zweite Leitung (22) zum Abführen des Plasmas (P) oder des angeregten Gasgemisches von der Abdeckung (37) für den zu behandelnden Wundbereich (13) zur Pumpe (36); und
einen piezoelektrischen Transformator (5) des Geräts (10) zur Erzeugung des Plasmas (P) oder des angeregten Gases bzw. Gasgemisches umfasst, wobei der piezoelektrische Transformator (5) im Gehäuse (30) untergebracht ist, ein Hochspannungsende (8) des piezoelektrischen Transformators (5) zu einer Öffnung (32) im Gehäuse (30) hin ausgerichtet ist und in einem Bereich der Öffnung (32) des Gehäuses (30) das Plasma (P) oder das angeregte Gas bzw. Gasgemisch erzeugbar ist.

2. Anordnung (1) nach Anspruch 1, wobei eine elektronische Kopplung (27) der Pumpe (36) mit dem Gerät (10) vorgesehen ist.

3. Anordnung (1) nach einem der vorangehenden Ansprüche, wobei mindestens ein Zuleitport (18) und mindestens ein Ableitport (19) an der Abdeckung (37) für den zu behandelnden Wundbereich (13) ausgebildet sind und die erste Leitung (21) für das Plasma (P) oder das angeregte Gas bzw. Gasgemisch mit dem Zuleitport (18) und die zweite Leitung (22) zu der Pumpe (36) mit dem Ableitport (19) verbunden sind.

4. Anordnung (1) nach einem der vorangehenden Ansprüche, wobei die erste Leitung (21) ein Anschlusselement (34) besitzt, das mit der Öffnung (32) des Gehäuses (30) lösbar verbindbar ist, so dass das vom Plasmaerzeugungsgerät (10) erzeugte Plasma (P) oder das angeregte Gas bzw. Gasgemisch in die erste Leitung (21) überführbar ist.

5. Anordnung (1) nach einem der vorangehenden Ansprüche, wobei die Abdeckung (37) mit einem umlaufenden Rand (11) den zu behandelnden Wundbereich (13) umschließt und mit einem Körperteil (40) verklebt bzw. verbunden ist.

6. Anordnung (1) nach einem der vorangehenden Ansprüche, wobei zwischen der Abdeckung (37) und dem zu behandelnden Wundbereich (13) eine Wundauflage (38) vorgesehen ist, wobei innerhalb der Wundauflage (38) ein Mittel (25) vorgesehen ist, mit dem eine Zwangsführung des Plasmas (P) oder des angeregten Gases bzw. Gasgemisches vom Zuleitport (18) zum Ableitport (19) bewirkbar ist und wobei das Plasma (P) auf einem Weg vom Zuleitport (18) zum Ableitport (19) über den zu behandelnden Wundbereich (13) geführt ist.

7. Anordnung (1) nach Anspruch 6, wobei das Mittel (25) zur Zwangsführung des Plasmas (P) oder des angeregten Gases bzw. Gasgemisches mehrere gerade Verteilkanäle (51) umfasst, die von einem Verteilelement (50) zu einem Sammelelement (59) führen, wobei das Verteilelement (50) mit dem Zuleitport (18) und das Sammelelement (59) mit dem Ableitport (19) verbunden ist.

8. Anordnung (1) nach Anspruch 6, wobei das Mittel (25) zur Zwangsführung des Plasmas (P) oder des angeregten Gases bzw. Gasgemisches ein einziger mäandernder Verteilkanal (52) ist, der direkt mit dem Zuleitport (18) und dem Ableitport (19) verbunden ist.

9. Anordnung (1) nach Anspruch 6, wobei das Mittel (25) zur Zwangsführung des Plasmas (P) oder des angeregten Gases bzw. Gasgemisches mehrere in einem regelmäßigen Gitter verteilte Strömungshindernisse (53) sind, die zwischen einem Verteilelement (50) und einem Sammelelement (59) angeordnet sind und wobei das Verteilelement (50) mit dem Zuleitport (18) und das Sammelelement (59) mit dem Ableitport (19) verbunden ist.

10. Anordnung (1) nach Anspruch 6, wobei das Mittel (25) zur Zwangsführung des Plasmas (P) oder des angeregten Gases bzw. Gasgemisches mehrere Verteilkanäle (54) mit jeweils zwei Umlenkungen (55) umfasst, die von einem Verteilelement (50) zu einem Sammelelement (59) führen, wobei das Verteilelement (50) mit dem Zuleitport (18) und das Sammelelement (59) mit dem Ableitport (19) verbunden ist.

11. Anordnung (1) nach Anspruch 6, wobei das Mittel (25) zur Zwangsführung des Plasmas (P) oder des angeregten Gases bzw. Gasgemisches eine zum zu behandelnden Wundbereich (13) offenporige Struktur ausgebildet hat.

12. Anordnung (1) nach einem der Ansprüche 7 bis 11, wobei die Verteilkanäle (51, 52, 54), die Strömungshindernisse (53) oder die offenporige Struktur hydrophob beschichtet sind.

13. Anordnung (1) nach einem der vorangehenden Ansprüche, wobei die Abdeckung (37) einen RFID-Chip (28) und / oder einen Farbsensor (29) umfasst.

14. Anordnung (1) nach einem der vorangehenden Ansprüche, wobei das Gerät (10) ein Handgerät ist.

## Claims

1. An assembly (1) for the treatment of wounds, comprising:
a device (10) for generating a plasma (P) or an excited gas or gas mixture;
a cover (37) for the wound region (13) to be treated;
a pump (36);
a housing (30) of the device (19);
**characterized in that** the assembly (1) comprises
a first line (21) for supplying the plasma (P) or the excited gas mixture from the device (10) to the cover (37) for the wound region (13) to be treated;
a second line (22) for discharging the plasma (P) or the excited gas mixture from the cover (37) for the wound region (13) to be treated to the pump (36); and
a piezoelectric transformer (5) of the device (10) for generating the plasma (P) or the excited gas or gas mixture;
wherein the piezoelectric transformer (5) is housed in the housing (30), a high-voltage end (8) of the piezoelectric transformer (5) is aligned toward an opening (32) in the housing (30), and the plasma (P) or the excited gas or gas mixture can be generated in a region of the opening (32) of the housing (30).

2. The assembly (1) according to Claim 1, wherein an electronic coupling (27) of the pump (36) with the device (10) is provided.

3. The assembly (1) according to any one of the preceding claims, wherein at least one supply port (18) and at least one discharge port (19) are formed on the cover (37) for the wound region (13) to be treated, and the first line (21) for the plasma (P) or the excited gas or gas mixture is connected to the supply port (18) and the second line (22) to the pump (36) is connected to the discharge port (19).

4. The assembly (1) according to any one of the preceding claims, wherein the first line (21) has a connection element (34), which is detachably connectable to the opening (32) of the housing (30), such that the plasma (P) generated by the plasma generating device (10) or the excited gas or gas mixture is transferable into the first line (21).

5. The assembly (1) according to any one of the preceding claims, wherein the cover (37) encloses the wound region (13) to be treated with a circumferential edge (11) and is adhesively bonded and/or connected to a body part (40).

6. The assembly (1) according to any one of the preceding claims, wherein a wound dressing (38) is provided between the cover (37) and the wound region (13) to be treated, wherein a means (25) is provided within the wound dressing (38), using which a forced guiding of the plasma (P) or the excited gas or gas mixture from the supply port (18) to the discharge port (19) can be caused, and wherein the plasma (P) is guided via the wound region (13) to be treated on a path from the supply port (18) to the discharge port (19).

7. The assembly (1) according to Claim 6, wherein the means (25) for the forced guiding of the plasma (P) or the excited gas or gas mixture comprises multiple linear distribution channels (51), which lead from a distribution element (50) to a collection element (59), wherein the distribution element (50) is connected to the supply port (18) and the collection element (59) is connected to the discharge port (19).

8. The assembly (1) according to Claim 6, wherein the means (25) for the forced guiding of the plasma (P) or the excited gas or gas mixture is a single meandering distribution channel (52), which is directly connected to the supply port (18) and the discharge port (19).

9. The assembly (1) according to Claim 6, wherein the means (25) for the forced guiding of the plasma (P) or the excited gas or gas mixture are multiple flow obstructions (53) distributed in a regular lattice, which are arranged between a distribution element (50) and a collection element (59), and wherein the distribution element (50) is connected to the supply port (18) and the collection element (59) is connected to the discharge port (19).

10. The assembly (1) according to Claim 6, wherein the means (25) for the forced guiding of the plasma (P) or the excited gas or gas mixture comprises a plurality of distribution channels (54) each having two deflections (55), which lead from a distribution element (50) to a collection element (59), wherein the distribution element (50) is connected to the supply port (18) and the collection element (59) is connected to the discharge port (19).

11. The assembly (1) according to Claim 6, wherein the means (25) for the forced guiding of the plasma (P) or the excited gas or gas mixture has formed an open-pored structure toward the wound region (13) to be treated.

12. The assembly (1) according to any one of Claims 7 to 11, wherein the distribution channels (51, 52, 54), the flow obstructions (53), or the open-pored structure have a hydrophobic coating.

13. The assembly (1) according to any one of the preceding claims, wherein the cover (37) comprises an RFID chip (28) and/or a color sensor (29).

14. The assembly (1) according to any one of the preceding claims, wherein the device (10) is a handheld device.

## Revendications

1. Disposition (1) pour le traitement d'une plaie comprenant
un appareil (10) destiné à produire un plasma (P) ou un gaz ou mélange de gaz excité,
une couverture (37) pour la zone de la plaie (13) à traiter,
une pompe (36),
un boîtier (30) de l'appareil (19),
**caractérisée en ce que** la Disposition (1) comprend
une première conduite (21) pour amener le plasma (P) ou le mélange gazeux excité de l'appareil (10) à la couverture (37) pour la zone de la plaie (13) à traiter,
une deuxième conduite (22) pour évacuer le plasma (P) ou le mélange gazeux excité de la couverture (37) pour la zone de la plaie (13) à traiter à la pompe (36), et
un transformateur piézoélectrique (5) de l'appareil (10) destiné à produire le plasma (P) ou le gaz ou mélange gazeux excité,
le transformateur piézoélectrique (5) étant logé dans le boîtier (30), une extrémité sous haute tension (8) du transformateur piézoélectrique (5) étant orientée vers une ouverture (32) dans le boîtier (30) et le plasma (P) ou le gaz ou mélange gazeux excité pouvant être produit au niveau de l'ouverture (32) du boîtier (30).

2. Disposition (1) selon la revendication 1, dans laquelle est prévu un couplage électronique (27) de la pompe (36) avec l'appareil (10).

3. Disposition (1) selon l'une des revendications précédentes, dans laquelle au moins un orifice d'arrivée (18) et au moins un orifice d'évacuation (19) sont formés sur la couverture (37) pour la zone de la plaie (13) à traiter, et la première conduite (21) pour le plasma (P) ou le gaz ou mélange gazeux excité est reliée à l'orifice d'arrivée (18) et la deuxième conduite (22) pour la pompe (36) est reliée à l'orifice d'évacuation (19).

4. Disposition (1) selon l'une des revendications précédentes, dans laquelle la première conduite (21) possède un élément de raccordement (34) qui peut être relié de façon amovible à l'ouverture (32) du boîtier (30), de sorte que le plasma (P) produit par le générateur de plasma (10) ou le gaz ou mélange gazeux excité peut être transféré dans la première conduite (21).

5. Disposition (1) selon l'une des revendications précédentes, dans laquelle la couverture (37) entoure avec un bord continu (11) la zone de la plaie (13) à traiter et est collée ou reliée à une partie du corps (40).

6. Disposition (1) selon l'une des revendications précédentes, dans laquelle une compresse (38) est prévue entre la couverture (37) et la zone de la plaie (13) à traiter, laquelle compresse (38) contient un moyen (25) qui permet d'obtenir un guidage forcé du plasma (P) ou du gaz ou mélange gazeux excité de l'orifice d'arrivée (18) à l'orifice d'évacuation (19) et dans laquelle le plasma (P) est guidé sur son trajet de l'orifice d'arrivée (18) à l'orifice d'évacuation (19) en passant pardessus la zone de la plaie (13) à traiter.

7. Disposition (1) selon la revendication 6, dans laquelle le moyen (25) pour le guidage forcé du plasma (P) ou du gaz ou mélange gazeux excité présente plusieurs canaux de distribution droits (51) menant d'un élément distributeur (50) à un élément collecteur (59), l'élément distributeur (50) étant relié à l'orifice d'arrivée (18) et l'élément collecteur (59) étant relié à l'orifice d'évacuation (19).

8. Disposition (1) selon la revendication 6, dans laquelle le moyen (25) pour le guidage forcé du plasma (P) ou du gaz ou mélange gazeux excité est un unique canal de distribution sinueux (52) relié directement à l'orifice d'arrivée (18) et à l'orifice d'évacuation (19).

9. Disposition (1) selon la revendication 6, dans laquelle le moyen (25) pour le guidage forcé du plasma (P) ou du gaz ou mélange gazeux excité est formé de plusieurs obstacles à l'écoulement (53) formant une grille régulière, qui sont disposés entre un élément distributeur (50) et un élément collecteur (59), et dans laquelle l'élément distributeur (50) est relié à l'orifice d'arrivée (18) et l'élément collecteur (59) est relié à l'orifice d'évacuation (19).

10. Disposition (1) selon la revendication 6, dans laquelle le moyen (25) pour le guidage forcé du plasma (P) ou du gaz ou mélange gazeux excité comprend plusieurs canaux de distribution (54) avec chacun deux déviations (55) qui mènent d'un élément distributeur (50) à un élément collecteur (59), l'élément distributeur (50) étant relié à l'orifice d'arrivée (18) et l'élément collecteur (59) étant relié à l'orifice d'évacuation (19).

11. Disposition (1) selon la revendication 6, dans laquelle le moyen (25) pour le guidage forcé du plasma (P) ou du gaz ou mélange gazeux excité possède une structure à pores ouverts vers la zone de la plaie (13) à traiter.

12. Disposition (1) selon l'une des revendications 7 à 11, dans laquelle les canaux de distribution (51, 52, 54), les obstacles à l'écoulement (53) ou la structure à pores ouverts sont dotés d'un revêtement hydrophobe.

13. Disposition (1) selon l'une des revendications précédentes, dans laquelle la couverture (37) comprend une puce RFID (28) et/ou un capteur de couleur (29).

14. Disposition (1) selon l'une des revendications précédentes, dans laquelle l'appareil (10) est un appareil manuel.
